# EUROPEAN PATENT SPECIFICATION

(11) **EP 3 001 965 B1**
(45) Date of publication and mention of the grant of the patent: **11.04.2018**
(21) Application number: 15187689.3
(22) Date of filing: 30.09.2015
(51) Int. Cl.: A61B 18/08, A61B 18/20, A61B 18/22, A61B 18/00, A61B 18/14, A61B 17/29

(54) **SURGICAL INSTRUMENTS WITH AN END-EFFECTOR ASSEMBLY INCLUDING OPTICAL FIBER FOR TREATING TISSUE**
CHIRURGISCHE INSTRUMENTE MIT EINER ENDEFFEKTORANORDNUNG MIT GLASFASER ZUR GEWEBEBEHANDLUNG
INSTRUMENTS CHIRURGICAUX AVEC UN ENSEMBLE D'EFFECTEUR TERMINAL COMPRENANT UNE FIBRE OPTIQUE POUR TRAITER UN TISSU

(30) Priority: 01.10.2014 US 201462058195 P; 22.09.2015 US 201514860786
(43) Date of publication of application: 06.04.2016
(60) Divisional application: 17186827.6
(73) Proprietor: Covidien LP, Mansfield, MA 02048 (US)
(72) Inventor: Jensen, Jeffrey L, Boulder, CO 80301 (US)
(74) Representative: Morgan, Marc

(56) References cited:
- US-A1- 2013 253 489
- US-A1- 2014 121 508
- US-A1- 2014 288 541
- US-B1- 6 221 069

## Description

### BACKGROUND

### 1. Technical Field

The present disclosure relates to surgical systems and devices for performing medical procedures. More particularly, the present disclosure relates to surgical systems and surgical instruments, such as, for example, vessel-sealing devices, with an end-effector assembly including longitudinal-firing optical fiber and/or transverse-firing optical fiber. The present disclosure also relates to methods of treating tissue using the surgical systems and instruments according to the present disclosure.

### 2. Discussion of Related Art

Electrosurgery involves the application of thermal and/or electrical energy to cut, dissect, ablate, coagulate, cauterize, seal, or otherwise treat tissue during a surgical procedure. Electrosurgery is typically performed using an electrosurgical generator operable to output electrical energy to an electrosurgical instrument adapted to transmit energy to a tissue site to be treated. Electrosurgical instruments, such as electrosurgical forceps having opposing jaw members, have come into widespread and accepted use in both open and minimally-invasive surgical procedures. By utilizing an electrosurgical forceps, a surgeon can cauterize, coagulate, desiccate and/or seal tissue and/or simply reduce or slow bleeding by controlling the intensity, frequency and duration of the electrosurgical energy applied through the jaw members to the tissue.

Thermal energy may also be applied using light energy using a laser or another light source. A laser is a device that emits light through a process of optical amplification based on the stimulated emission of electromagnetic radiation. A laser may be classified as operating in either continuous or pulsed mode, depending on whether the power output is essentially continuous over time or whether its output takes the form of pulses of light.

Flexible optical fibers may be used to deliver laser light from a laser device to a treatment site. The delivery end of the fiber may be encased in a metal sheathed probe having a design which is optimized for a particular procedure. A common laser-based surgical procedure is holmium laser enucleation of the prostate (HoLEP). In this procedure, a holmium:yttrium aluminium garnet (Ho:YAG) laser is used to remove obstructive prostate tissue. Laser fibers have also been used for incision, necrosis, excision, and cauterization of tissue.

There are a number of design considerations in the construction of optical fibers for medical applications, such as, for example, sterilizability, quartz core integrity and purity, power capacity, and index of refraction of materials. A quartz, plastic, or silicone cladding may be used to constrain the laser light to the quartz core. The radiant energy beam transmission is made possible because photons are constrained to the core of the fiber due to internal reflectance by the quartz cladding interface.

A variety of types of laser systems have been designed for operation in a wide range of applications related to surgical procedures, e.g., surgical ablation, vaporization, incision, excision, coagulation and cauterization of tissue, and other medical procedures. These procedures may be performed in air or in fluid, either in open or in endoscopic methods, through natural body channels or through incisions. There is a need for surgical instruments capable of performing multiple procedures and modalities. Relevant prior art is known e.g. from US 2013/253489 A1, US 6221069 B1 and US 2014/121508 A1.

### SUMMARY

The present disclosure provides a surgical instrument capable of tissue dissection, tissue transection, ligation of vessels, and hemostasis. The surgical instrument is configured for forming otomies or colpotomies, tissue scoring, dissection of tissue with or without hemostasis, coagulation, fulguration, and the like using electrosurgical and light energy.

According to an aspect of the present disclosure, an end-effector assembly is provided. The end-effector assembly includes first and second jaw members, a first outer housing associated with the first jaw member, and a second outer housing associated with the second jaw member. Each of the first and second jaw members includes a sealing plate. One or both of the first and second jaw members is movable from a first position wherein the jaw members are disposed in spaced relation relative to one another to a second position wherein the sealing plates cooperate to grasp tissue therebetween. One or more longitudinal-firing optical fibers are associated with either one or both of the first and second outer housings. One or more transverse-firing optical fibers are associated with either one or both of the first and second jaw members.

According to an aspect of the present disclosure, a forceps is provided. The forceps includes a housing, a shaft including a distal end and a proximal end operatively coupled to the housing, and an end-effector assembly coupled to the distal end of the shaft and including first and second jaw members. Each of the first and second jaw members includes a sealing plate. One or both of the first and second jaw members is movable from a first position wherein the jaw members are disposed in spaced relation relative to one another to a second position wherein the sealing plates cooperate to grasp tissue therebetween. The forceps also includes a longitudinal-firing optical fiber operably coupled to one of the first and second jaw members and configured to emit light into tissue.

According to another aspect of the present disclosure, a method of treating tissue is provided. The method includes positioning an end-effector assembly including first and second jaw members at a first position within tissue. Each of the first and second jaw members includes a sealing plate. One or both of the first and second jaw members is movable from a first position wherein the jaw members are disposed in spaced relation relative to one another to a second position wherein the sealing plates cooperate to grasp tissue therebetween. The method also includes activating a transverse-firing optical fiber associated with one or both of the first and second jaw members to emit light into tissue grasped between the sealing plates. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

### BRIEF DESCRIPTION OF THE DRAWINGS

Objects and features of the presently-disclosed end-effector assemblies including longitudinal-firing optical fiber and/or transverse-firing optical fiber for use in surgical instruments, systems including the same, and methods of treating tissue using the same of the present disclosure will become apparent to those of ordinary skill in the art when descriptions of various embodiments thereof are read with reference to the accompanying drawings, of which:
FIG. 1 is a perspective view of an endoscopic bipolar forceps in accordance with an embodiment of the present disclosure;
FIG. 2 is a perspective view of an open surgical forceps in accordance with an embodiment of the present disclosure;
FIG. 3 is an enlarged, perspective view of an end-effector assembly that includes a longitudinal-firing optical fiber and a transverse-firing optical fiber in accordance with an embodiment of the present disclosure;
FIG. 4 is an enlarged, perspective view of an end-effector assembly that includes a longitudinal-firing optical fiber in accordance with the present disclosure;
FIG. 5 is an enlarged, perspective view of an end-effector assembly that includes a transverse-firing optical fiber in accordance with the present disclosure;
FIG. 6 is an enlarged, perspective view of a distal portion of the shaft and the end-effector assembly of the endoscopic bipolar forceps shown in FIG. 1 in accordance with the present disclosure; and
FIG. 7 is a schematic diagram of a robotic surgery system in accordance with the present disclosure.

### DETAILED DESCRIPTION

Embodiments of end-effector assemblies including a longitudinal-firing optical fiber and/or a transverse-firing optical fiber for use in surgical instruments, systems including the same, and methods of treating tissue using the surgical instruments of the present disclosure are described with reference to the accompanying drawings. Like reference numerals may refer to similar or identical elements throughout the description of the figures. As shown in the drawings and as used in this description, when referring to relative positioning on an object, the term "proximal" is used to refer to a portion of the apparatus, or component thereof, closer to the user and the term "distal" is used to refer to a portion of the apparatus, or component thereof, farther from the user.

This description may use the phrases "in an embodiment," "in embodiments," "in some embodiments," or "in other embodiments," which may each refer to one or more of the same or different embodiments in accordance with the present disclosure.

As it is used in this description, "transverse-firing" denotes a laser fiber that has the capability to emit light (e.g., high-intensity visible or ultraviolet electromagnetic (EM) energy produced by a laser or other high-intensity EM energy source) in a direction away from a longitudinal axis defined by the laser. The terms "end-firing" and "longitudinal-firing" as used herein denote a laser fiber that has the capability to emit light along a longitudinal axis of the fiber. For the purposes of this description, the terms "laser fiber" and "optical fiber" are used interchangeably.

As it is used in this description, "transmission line" generally refers to any transmission medium that can be used for the propagation of signals from one point to another. As it is used in this description, "switch" or "switches" generally refers to any hardware or software-based actuators, mechanical actuators, electro-mechanical actuators, such as rotatable actuators, pivotable actuators, toggle-like actuators, buttons, etc., optical actuators, or any suitable device that generally fulfills the purpose of connecting and disconnecting electronic devices, or components thereof, instruments, equipment, transmission lines or connections and appurtenances thereto.

Various examples of the present disclosure provide end-effector assemblies including an optical fiber of the transverse-firing type wherein light exits the fiber transverse to the longitudinal axis defined by the optical fiber. In embodiments, end-effector assemblies may include an optical fiber of the longitudinal-firing type. Embodiments of the presently-disclosed end-effector assembly may be configured to emit light in the form of optical pulses, continuous-wave laser irradiation, and/or other forms of light energy.

Various embodiments of the present disclosure provide electrosurgical instruments with an end-effector assembly including a longitudinal-firing optical fiber and a transverse-firing optical fiber. Embodiments of the presently-disclosed electrosurgical instruments may be configured to provide monopolar electrosurgical energy and/or bipolar electrosurgical energy, which may be suitable for sealing, cauterizing, coagulating, desiccating, and/or cutting tissue, e.g., vessels and vascular tissue. Embodiments of the presently-disclosed electrosurgical instruments may be suitable for utilization in endoscopic surgical procedures and/or suitable for utilization in open surgical applications.

Embodiments of the presently-disclosed electrosurgical instruments may be implemented using a variety of types of energy, e.g., electrosurgical energy at radio frequencies (RF) and/or at other frequencies, optical, and/or thermal energy. Embodiments of the presently-disclosed electrosurgical instruments may be configured to be connectable to one or more energy sources, e.g., laser sources, RF generators, and/or self-contained power sources. Embodiments of the presently-disclosed electrosurgical instruments may be connected through a suitable bipolar transmission line and/or other transmission lines to an electrosurgical generator and/or other suitable energy source.

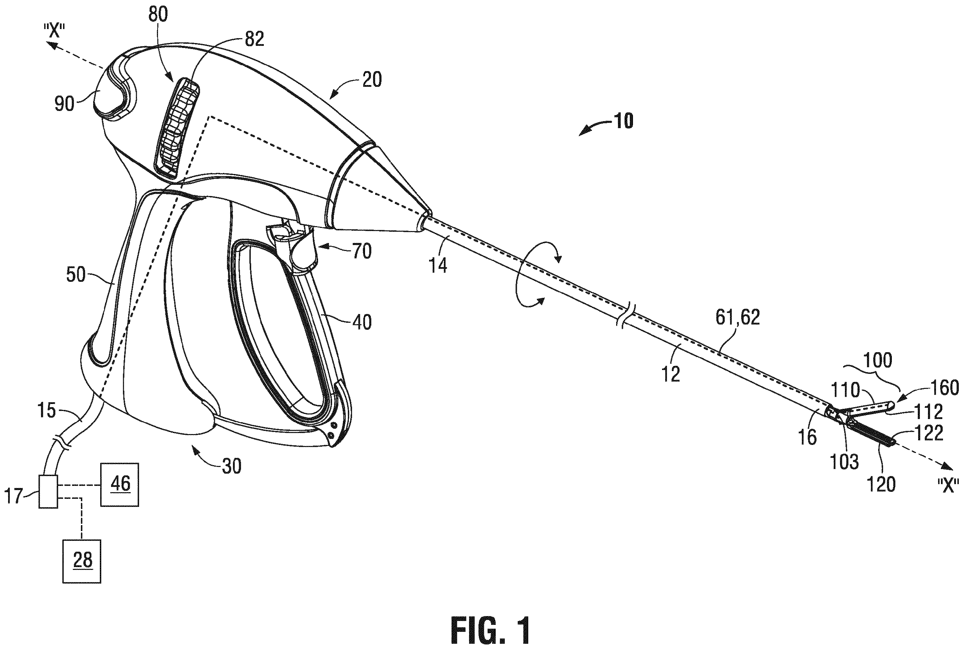

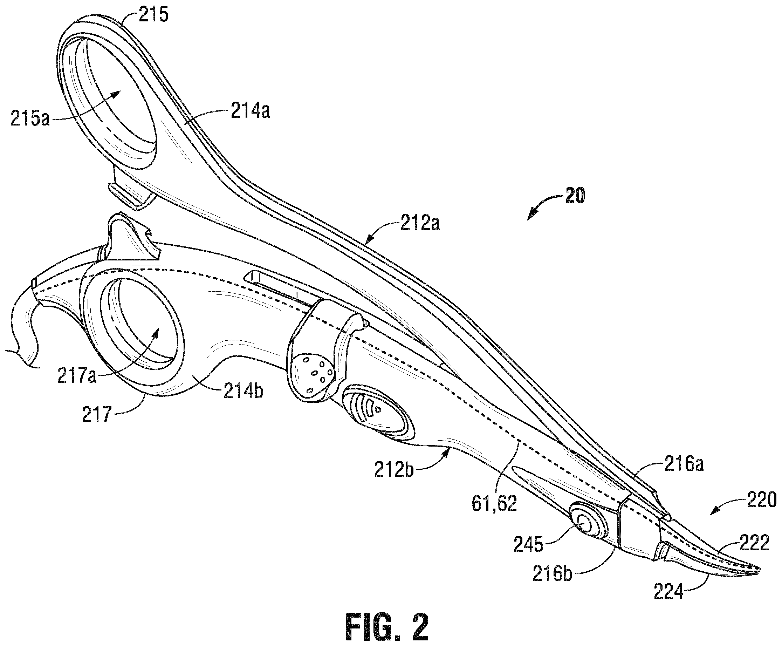

FIG. 1 depicts a bipolar forceps 10 for use in connection with endoscopic surgical procedures, and an open version of a bipolar forceps 20 is shown in FIG. 2. Although the following description describes the use of a bipolar forceps, the teachings of the present disclosure may also apply to a variety of surgical instruments.

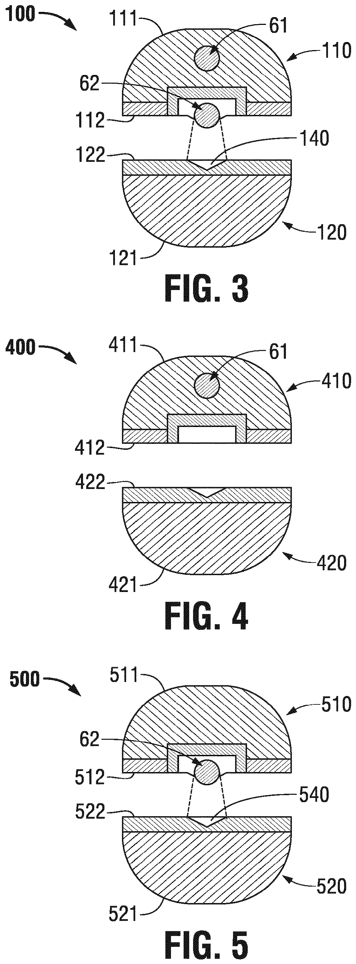

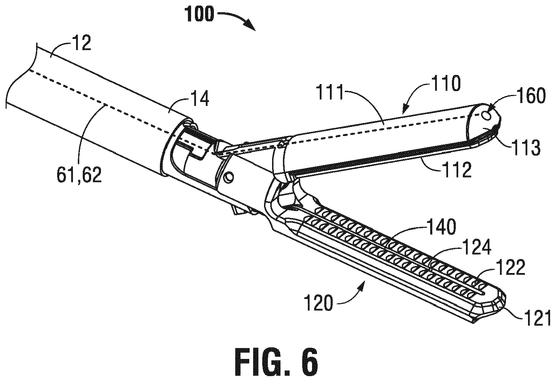

With reference to FIG. 1, the forceps 10 includes a housing 20, a handle assembly 30, a rotatable assembly 80, activation switch 70, and an end-effector assembly 100 that includes a longitudinal-firing optical fiber 61 and a transverse-firing optical fiber 62. An embodiment of the end-effector assembly 100 of FIG. 1 is shown in more detail in FIGS. 3 and 6. In embodiments, other end-effector assemblies such as, the end-effector assembly 400 shown in FIG. 4 and the end-effector assembly 500 shown in FIG. 5, may also be used. One or more components of the forceps 10, e.g., the housing 20, the rotation assembly 80, the activation switch 70, and/or the end-effector assembly 100, may be adapted to mutually cooperate to grasp, seal and/or divide tissue, e.g., tubular vessels and vascular tissue. Forceps 10 may include additional, fewer, or different components than shown in FIG. 1, depending upon a particular purpose or to achieve a desired result.

Forceps 10 includes an elongated shaft 50 having a distal end 16 configured to mechanically engage the end-effector assembly 100. End-effector assembly 100 includes two jaw members 110 and 120 disposed in opposing relation relative to one another. Shaft 50 extends from the housing 20 and supports movement of other components therethrough, e.g., to impart movement to the upper jaw member 110 and/or the lower jaw member 120. Rotation assembly 80 is operably coupled to the shaft 50 and is rotatable about a longitudinal axis "X - X" defined by the shaft 50. The proximal end 14 of the shaft 50 is received within the housing 20 or is otherwise engaged to the housing 20, and connections relating thereto are disclosed in commonly-assigned U.S. Patent No. 7,156,846 entitled "Vessel Sealer And Divider For Use With Small Trocars And Cannulas," commonly-assigned U.S. Patent No. 7,597,693 entitled "Vessel Sealer And Divider For Use With Small Trocars And Cannulas" and commonly-assigned U.S. Patent No. 7,771,425 entitled "Vessel Sealer And Divider Having A Variable Jaw Clamping Mechanism," the entire contents of all of which are incorporated by reference herein.

Activation switch 70 is configured to facilitate the transmission of the energy from one or more energy sources including a laser source 46 and/or electrosurgical generator 28, to the end-effector assembly 100. As an alternative to, or in addition to, the activation switch 70, forceps 10 may include any other suitable inputs, such as touch screen, virtual/projected interfaces, voice input technology, and the like.

In some examples, activation switch 70 is configured as a two-stage switch wherein a first stage of the switch 70 effects a first operational mode of two or more operational modes and a second stage of the switch 70 effects a second operational mode that is different from the first operational mode. In some examples, in the first operational mode, energy is provided for sealing, and thus energy is supplied from the electrosurgical generator 28 to seal tissue as described in further detail below. In the second operational mode, energy is provided for scoring or dissecting tissue using light energy, and thus, energy is supplied from the laser source 46 to the transverse-firing optical fiber 62 to treat, e.g., seal or cut, tissue as described in further detail below.

Switch 70 may also have a variable resistance such that the first stage occurs when a first depression force is applied to the switch 70, and the second stage occurs when a second depression force greater than the first depression force is applied to the switch 70. The first depression force and/or the second depression force may cause electrical contacts within the switch 70 to close, thereby completing a circuit between the end-effector assembly 100 and an energy source, e.g., electrosurgical generator 28 and laser source 46, respectively. In other examples, operation of the switch 70 may include opening electrical contacts or processor-controlled power delivery that receives information from the switch 70 and directs a corresponding circuit reaction based on the information. In some examples, when a first depression force is applied to the switch 70, the forceps 10 transitions to the first operational mode, e.g., energy is imparted to the sealing plates 112 and 122 of the jaw members 110 and 120. In some examples, when a second depression force is applied to the switch 70, the forceps 10 transitions to the second operational mode, e.g., energy is imparted to the transverse-firing optical fiber 62.

Forceps 10 also includes a transmission line 15, which may connect directly to an electrosurgical generator 28 and the laser source 46. Transmission line 15 may be formed from a suitable flexible, semi-rigid, or rigid transmission line. In some examples, the transmission line 15 connects the forceps 10 to a connector 17, which further operably connects the forceps 10 to the electrosurgical generator 28, and which further connects the forceps 10 to the laser light source 46. Transmission line 15 may be internally divided into one or more transmission line leads each of which transmits energy through their respective feed paths to the end-effector assembly 100. Transmission line 15 may include an optical fiber (e.g., optical fiber 61, 62) configured to transmit light to the end-effector assembly 100.

Laser source 46 may include any laser suitable for use with surgical devices. In some examples, the laser source 46 may include an ultraviolet laser, an ultraviolet infrared laser, a pulsed laser, a gas laser, a solid-state laser, a diode laser, an infrared pulsed diode laser, a Holmium:YAG (Ho:YAG) laser, a neodymium-doped:YAG (Nd:YAG) laser, a semiconductor laser diode, and/or a potassium-titanyl phosphate crystal (KTP) laser. In some examples, more than one laser may be included in the laser source 46, and more than one laser may be used during a surgical procedure. Laser source 46 may include a processor (not shown) configured to provide timing, wavelength, and/or power control of the one or more lasers. In further examples, the laser source 46 may include mechanisms for laser selection, filtering, temperature compensation, and/or Q-switching operations. In yet further examples, the laser source 46 may include a function generator and optical shutter used to modulate a continuous-wave laser to generate pulsed output.

Electrosurgical generator 28 may be any generator suitable for use with surgical devices, and may be configured to provide various frequencies of electromagnetic energy. Forceps 10 may alternatively be configured as a wireless device or battery-powered.

With reference to FIGS. 1 and 6, the end-effector assembly 100 is configured as a unilateral assembly that includes a stationary jaw member 120 mounted in fixed relation to the shaft 50 and a pivoting jaw member 110 movably mounted about a pivot pin 103 coupled to the stationary jaw member 120. Jaw members 110 and 120 may be curved at various angles to facilitate manipulation of tissue and/or to provide enhanced line-of-sight for accessing targeted tissues. End-effector assembly 100 may include one or more electrically-insulative elements to electrically isolate the pivoting jaw member 110 (also referred to herein as "first jaw member 110") from the stationary jaw member 120 (also referred to herein as "second jaw member 120") and/or to isolate both or one of the jaw members 110 and 120 from the shaft 50. Alternatively, the forceps 10 may include a bilateral assembly, i.e., both jaw members 110 and 120 are movable relative to one another and are pivotable about an axis defined by a pivot pin.

Jaw members 110 and 120, as shown in FIGS. 3 and 6, include an outer housing 111 and 121 and electrically-conductive, tissue-engaging surface or sealing plate 112 and 122, respectively. The sealing plates 112 and 122 are arranged in opposed relation relative to one another and are associated with respective outer housing 111 and 121. In some embodiments, the outer housings 111 and 121 define a cavity therein configured to at least partially encapsulate and/or securely engage the sealing plates 112 and 122, respectively, and/or other jaw member components. As described in more detail below, the longitudinal-firing optical fiber 61 is associated with the outer housing 111 and/or the cavity defined therein. The outer housings 111 and 121 may be formed, at least in part, of an electrically non-conductive or substantially electrically non-conductive material. In some embodiments, the outer housing 111 and 121 may include ceramic or any of a variety of suitable non-electrically conductive materials such as polymeric materials, e.g., plastics, and/or other insulative materials. In some embodiments, as shown for example in FIG. 6, the distal end 160 of the longitudinal-firing optical fiber 61 is disposed at the distal end of the outer housing 111 of the first jaw member 110.

As depicted in FIG. 1, the end-effector assembly 100 is rotatable in either direction about the longitudinal axis "X - X" through rotation, either manually or otherwise, of the rotatable assembly 80. Rotatable assembly 80 generally includes two halves (not shown), which, when assembled about a tube of shaft 12, form a generally circular rotatable member 82. Rotatable assembly 80, or portions thereof, may be configured to house a drive assembly (not shown), or components thereof. A reciprocating sleeve (not shown) is slidingly disposed within the shaft 12 and remotely operable by the drive assembly (not shown). Examples of rotatable assembly examples and drive assembly examples of the forceps 10 are described in the above-mentioned, commonly-assigned U.S. Patent Nos. 7,156,846, 7,597,693 and 7,771,425.

Handle assembly 30 includes a fixed handle 50 and a movable handle 40. In some examples, the fixed handle 50 is integrally associated with the housing 20, and the movable handle 40 is selectively movable relative to the fixed handle 50. Movable handle 40 of the handle assembly 30 is connected to the drive assembly (not shown). Movement of the movable handle 40 toward the fixed handle 50 pulls the drive assembly (not shown) proximally to impart movement to the jaw members 110 and 120 from an open position, wherein the jaw members 110 and 120 are disposed in spaced relation relative to one another, to a clamping or closed position, wherein the jaw members 110 and 120 cooperate to grasp tissue therebetween. Examples of handle assemblies of the forceps 10 are described in the above-mentioned, commonly-assigned U.S. Patent Nos. 7,156,846, 7,597,693 and 7,771,425.

Forceps 10 includes a switch 90 configured to permit the user to selectively activate the laser source 46 to provide light to the longitudinal-firing optical fiber 61. Although FIG. 1 depicts the switch 90 disposed at the proximal end of the housing assembly 20, switch 90 may be disposed on another part of the forceps 10 (e.g., the fixed handle 50, rotatable member 82, etc.) or another location on the housing assembly 20. In examples, any of the switches, such as the switch 90 and the switch 70, may be configured to effect any of the operational modes, namely, energize the optical fiber 61, energize the optical fiber 62, and energize sealing plates 112 and 122.

In FIG. 2, an embodiment of an open forceps 20 is shown for use with various surgical procedures and generally includes a pair of opposing shafts 212a and 212b having an end-effector assembly 220 attached to the distal ends 216a and 216b thereof, respectively. End-effector assembly 220 includes a pair of opposing jaw members 222 and 224 that are pivotably connected about a pivot pin 265 and movable relative to one another to grasp tissue. Forceps 20 includes optical fiber 61, 62 associated with at least one of the shafts, e.g., shaft 212b, suitable for transmitting energy from one or more energy sources (e.g., laser source 46 shown in FIG. 1) to the end-effector assembly 220. The open forceps 20 may include any number of switches that are also configured to effect any of the operational modes, namely, energize the optical fiber 61, energize the optical fiber 62, and energize sealing plates (not shown) of the jaw members 222 and 224, as described above with respect to forceps 10.

Each shaft 212a and 212b includes a handle 215 and 217, respectively, disposed at the proximal end 214a and 214b thereof, respectively. Each handle 215 and 217 defines a finger and/or thumb hole 215a and 217a, respectively, therethrough for receiving the user's finger or thumb. Finger and/or thumb holes 215a and 217a facilitate movement of the shafts 212a and 212b relative to one another to pivot the jaw members 222 and 224 from an open position, wherein the jaw members 222 and 224 are disposed in spaced relation relative to one another, to a clamping or closed position, wherein the jaw members 222 and 224 cooperate to grasp tissue therebetween. End-effector assembly 220 may include any feature or combination of features of the longitudinal-firing and transverse-firing optical fibers 61, 62 disclosed herein with respect to forceps 10.

FIGS. 3-5 illustrate various embodiments of longitudinal-firing and transverse-firing optical fibers 61, 62 with reference to forceps 10 and/or forceps 20. FIG. 3 shows the end-effector assembly 100 of the forceps 10 shown in FIG. 1, including the first and second jaw members 110 and 120. As depicted in FIG. 3, the end-effector assembly 100 includes a longitudinal-firing optical fiber 61 and a transverse-firing optical fiber 62. Longitudinal-firing optical fiber 61 may be used to emit light along the longitudinal axis "X-X." This allows for treatment of tissue disposed in front of the jaw members 110 and 120 and may be used to create otomies or colpotomies, for tissue scoring, for dissection of tissue with and without hemostasis, and coagulation and fulguration. Transverse-firing optical fiber 62 may be used for tissue dissection, tissue transection, ligation of vessels, all with or without hemostasis, and other procedures for tissue disposed between the jaw members 110 and 120.

Various parameters of EM energy delivered to the longitudinal-firing optical fiber 61 and/or the transverse-firing optical fiber 62 may be controlled to adjust rate of treatment, depth of tissue lesions, extent of hemostasis, thermal spread, and other tissue effects. Adjustable EM energy parameters include, but are not limited to the pulse duration and duty cycle, power, width, rise and fall time, wavelength, focus, penetration depth, repetition rate and combinations thereof.

In some examples, the jaw member 120 includes a reflective groove 140. Reflective groove 140 may be made from a polished metal or a coating may be applied to the jaw member 120 if the jaw member 120 is formed from a non-metal and/or nonreflective material (e.g., plastic). Reflective groove 140 is configured to reflect laser light back through the tissue.

In some examples, end-effector assembly 100 may include a laser emitter (not shown) coupled to the distal end of the longitudinal-firing optical fiber 61. The laser emitter may have any suitable shape for transmitting and/or focusing light energy including, but not limited to, conical, frustoconical, pyramidal, cylindrical, any other granulated surfaced, and combinations thereof.

FIG. 4 shows an end-effector assembly 400 for use with endoscopic surgical procedures. End-effector assembly 400 includes opposing jaw members 410 and 420 which cooperate to effectively grasp tissue therebetween, e.g., for sealing and/or cutting purposes. In some embodiments, as shown in FIG. 4, each of the jaw members 410 and 420 includes an outer housing 411 and 421 and an electrically-conductive tissue-engaging surface or sealing plate 412 and 422, respectively. The outer housings 411 and 421 and the sealing plates 412 and 422 shown in FIG. 4 are similar to the outer housings 111 and 121 and the sealing plates 112 and 122, respectively, shown in FIGS. 1 and 3, and further description of the like elements is omitted in the interests of brevity. One or more of the longitudinal-firing optical fiber 61 discussed above with respect to FIG. 3 may be associated with the outer housing 411 (and/or outer housing 421).

FIG. 5 shows an end-effector assembly 500 for use with endoscopic surgical procedures. End-effector assembly 500 includes opposing jaw members 510 and 520 which cooperate to effectively grasp tissue therebetween, e.g., for sealing and/or cutting purposes. Each of the jaw members 510 and 520 includes an outer housing 511 and 521 and an electrically-conductive tissue-engaging surface or sealing plate 512 and 422, respectively. The outer housings 511 and 521 and the sealing plates 512 and 522 shown in FIG. 4 are similar to the outer housings 111 and 121 and the sealing plates 112 and 122, respectively, shown in FIG. 1, and further description of the like elements is omitted in the interests of brevity.

One or more of the transverse-firing optical fibers 62, for example discussed above with respect to FIG. 3, may be associated with any of the various components of the jaw member 510 (and/or the jaw member 520). In some examples, the jaw member 520 includes a reflective groove 540. Reflective groove 540 is configured to reflect laser light back through the tissue.

A method of treating tissue in accordance with the present disclosure includes positioning the end-effector assembly 100 including first and second jaw members 110 and 120 at a first position relative to tissue. One or both of the first and second jaw members 110 and 120 are moved from a first position wherein the jaw members 110 and 120 are disposed in spaced relation relative to one another to a second position wherein the sealing plates 112 and 122 cooperate to grasp tissue therebetween. Thereafter, the sealing plates 112 and 122 are energized to seal tissue. Alternatively, a transverse-firing optical fiber 62 associated with one or both of the first and second jaw members 110 and 120 is activated to emit light into tissue grasped between the sealing plates 112 and 122 to seal and/or cut tissue. In examples, sealing of tissue may be activated by the switch 70 by actuating the switch 70 into the first operational mode and cutting may be accomplished by actuating the switch 70 into the second operational mode.

In some examples, activating the transverse-firing optical fiber 62 includes emitting light in the form of optical pulses. Activating the transverse-firing optical fiber 62 may additionally, or alternatively, include emitting light in the form of continuous-wave laser irradiation. In some examples, activating the transverse-firing optical fiber includes the transverse-firing optical fiber emitting light to provide dissection, ligation and transection of tissue.

In some examples, one or both of the first and second jaw members 110 and 120 may include a longitudinal-firing optical fiber 61. Positioning the end-effector assembly may include activating the longitudinal-firing optical fiber 61 operably coupled to at least one of the first and second jaw members 110 and 120. The method of treating tissue may also include activating the longitudinal-firing optical fiber 61 operably coupled to at least one of the first and second jaw members to emit light into tissue to create otomies and/or colpotomies. In embodiments, the longitudinal-firing optical fiber 61 may be activated by actuating the switch 90.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

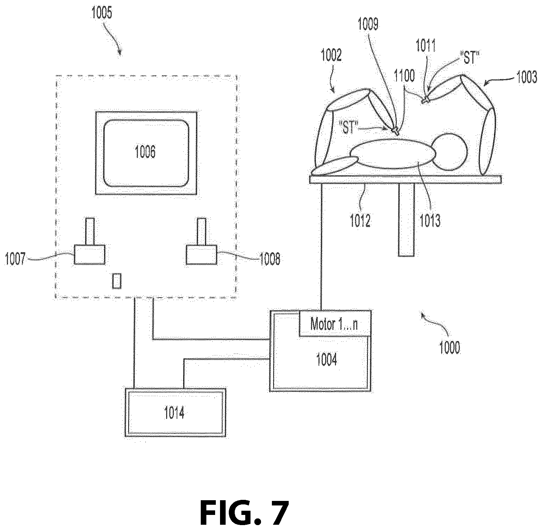

Referring initially to FIG. 7, a medical work station is shown generally as work station 1000 and generally may include a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100, in accordance with any one of several embodiments disclosed herein, as will be described in greater detail below.

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool (including end effector 1100) execute a desired movement according to a movement defined by means of manual input devices 1007, 1008. Control device 1004 may also be set up in such a way that it regulates the movement of robot arms 1002, 1003 and/or of the drives.

Medical work station 1000 may be configured for use on a patient 1013 lying on a patient table 1012 to be treated in a minimally invasive manner by means of end effector 1100. Medical work station 1000 may also include more than two robot arms 1002, 1003, the additional robot arms likewise being connected to control device 1004 and being telemanipulatable by means of operating console 1005. A medical instrument or surgical tool (including an end effector 1100) may also be attached to the additional robot arm. Medical work station 1000 may include a database 1014, in particular coupled to with control device 1004, in which are stored, for example, preoperative data from patient/living being 1013 and/or anatomical atlases.

The above-described end-effector examples including any combination of features of the presently-disclosed jaw members including longitudinal-firing optical fiber and/or transverse-firing optical fiber configured to emit light may be used in connection with jaw members of varied geometries, e.g., lengths and curvatures, such that variously-configured jaw members may be fabricated and assembled into various end-effector configurations depending upon a particular purpose.

The above-described bipolar forceps embodiments may be suitable for utilization with endoscopic surgical procedures and/or hand-assisted, endoscopic and laparoscopic surgical procedures. The above-described bipolar forceps embodiments may be suitable for utilization in open surgical applications.

Although embodiments have been described in detail with reference to the accompanying drawings for the purpose of illustration and description, it is to be understood that the disclosed processes and apparatus are not to be construed as limited thereby. It will be apparent to those of ordinary skill in the art that various modifications to the foregoing embodiments may be made without departing from the scope of the disclosure.

## Claims

1. An end-effector assembly (100, 400, 500), comprising:
first and second jaw members (110, 120, 410, 420, 510, 520), each of the first and second jaw members including a sealing plate (112, 122, 412, 422, 512, 522), at least one of the first and second jaw members movable from a first position wherein the jaw members are disposed in spaced relation relative to one another to a second position wherein the sealing plates cooperate to grasp tissue therebetween;
at least one longitudinal-firing optical fiber (61) disposed in at least one of the first jaw member or the second jaw member; and
at least one transverse-firing optical fiber (62) disposed in at least one of the first jaw member or the second jaw member,
wherein the at least one longitudinal-firing optical fiber is configured for treatment of tissue disposed in front of the jaw members and the at least one transverse-firing optical fiber is configured for treatment of tissue disposed between the first and second jaw members.

2. The end-effector assembly of claim 1, wherein the longitudinal-firing optical fiber is configured to output light along a longitudinal axis defined by the longitudinal-firing optical fiber.

3. The end-effector assembly of claim 1 or claim 2, wherein the transverse-firing optical fiber is configured to output light along a plane perpendicular to a longitudinal axis defined by the transverse-firing optical fiber.

4. The end effector of claim 1, 2 or 3, wherein each of the first and second jaw members further includes an outer housing (111, 121, 411, 421, 511, 521).

5. The end effector of claim 4, wherein the longitudinal-firing optical fiber is disposed at a distal end of one of the outer housings such that a distal end of the longitudinal-firing optical fiber is exposed through one of the outer housings.

6. The end effector of claim 1 wherein the sealing plates are electrically conductive and configured for bipolar energization to seal tissue.

## Patentansprüche

1. Endeffektoranordnung (100, 400, 500), umfassend:
ein erstes und ein zweites Klemmbackenelement (110, 120, 410, 420, 510, 520), wobei jedes von dem ersten und dem zweiten Klemmbackenelement eine Dichtungsplatte (112, 122, 412, 422, 512, 522) aufweist, wobei zumindest eines von dem ersten und dem zweiten Klemmbackenelement von einer ersten Position, wobei die Klemmbackenelemente in beabstandeter Beziehung zueinander angeordnet sind, zu einer zweiten Position, wobei die Dichtungsplatten zusammenarbeiten, um Gewebe dazwischen zu greifen, beweglich sind;
zumindest eine in Längsrichtung feuernde optische Faser (61), die in zumindest einem von dem ersten oder dem zweiten Klemmbackenelement angeordnet ist; und
zumindest eine in transversaler Richtung feuernde optische Faser (62), die in zumindest einem von dem ersten oder dem zweiten Klemmbackenelement angeordnet ist,
wobei die zumindest eine in Längsrichtung feuernde optische Faser zur Behandlung von Gewebe, das vor den Klemmbackenelementen angeordnet ist, konfiguriert ist und wobei die zumindest eine in transversaler Richtung feuernde optische Faser zur Behandlung von Gewebe, das zwischen dem ersten und dem zweiten Klemmbackenelement angeordnet ist, konfiguriert ist.

2. Endeffektoranordnung nach Anspruch 1, wobei die in Längsrichtung feuernde optische Faser konfiguriert ist, um Licht entlang einer Längsachse, die durch die in Längsrichtung feuernde optische Faser definiert ist, auszugeben.

3. Endeffektoranordnung nach Anspruch 1 oder Anspruch 2, wobei die in transversaler Richtung feuernde optische Faser konfiguriert ist, um Licht entlang einer Ebene senkrecht zu einer Längsachse, die durch die in transversaler Richtung feuernde optische Faser definiert ist, auszugeben.

4. Endeffektoranordnung nach Anspruch 1, 2 oder 3, wobei jeder von dem ersten und dem zweiten Klemmbackenelement ein äußeres Gehäuse (111, 121, 411, 421, 511, 521) aufweist.

5. Endeffektoranordnung nach Anspruch 4, wobei die in Längsrichtung feuernde optische Faser an einem distalen Ende von einem der äußeren Gehäuse angeordnet ist, sodass ein distales Ende der in Längsrichtung feuernden optischen Faser durch eines der äußeren Gehäuse exponiert ist.

6. Endeffektoranordnung nach Anspruch 1, wobei die Dichtungsplatten elektrisch leitend sind und zur bipolaren Erregung konfiguriert sind, um Gewebe abzudichten.

## Revendications

1. Ensemble effecteur terminal (100, 400, 500) comprenant :
un premier et un second élément de mors (110, 120, 410, 420, 510, 520), chacun des premier et second éléments de mors comprenant une plaque d'étanchéité (112, 122, 412, 422, 512, 522), au moins l'un des premier et second éléments de mors pouvant se déplacer d'une première position, dans laquelle les éléments de mors sont disposés espacés l'un par rapport à l'autre, à une seconde position, dans laquelle les plaques d'étanchéité coopèrent pour saisir un tissu entre elles ;
au moins une fibre optique à déclenchement longitudinal (61) disposée dans au moins l'un du premier élément de mors ou du second élément de mors ; et
au moins une fibre optique à déclenchement transversal (62) disposée dans au moins l'un du premier élément de mors ou du second élément de mors, dans lequel la au moins une fibre optique à déclenchement longitudinal est configurée pour traiter un tissu disposé devant les éléments de mors et la au moins une fibre optique à déclenchement transversal est configurée pour le traitement d'un tissu disposé entre le premier et le second élément de mors.

2. Ensemble effecteur terminal selon la revendication 1, dans lequel la fibre optique à déclenchement longitudinal est configurée pour délivrer de la lumière le long d'un axe longitudinal défini par la fibre optique à déclenchement longitudinal.

3. Ensemble effecteur terminal selon la revendication 1 ou la revendication 2, dans lequel la fibre optique à déclenchement transversal est configurée pour délivrer de la lumière le long d'un plan perpendiculaire à un axe longitudinal défini par la fibre optique à déclenchement transversal.

4. Effecteur terminal selon la revendication 1, 2 ou 3, dans lequel chacun des premier et second éléments de mors comprend en outre un boîtier externe (111, 121, 411, 421, 511, 521).

5. Effecteur terminal selon la revendication 4, dans lequel la fibre optique à déclenchement longitudinal est disposée à une extrémité distale de l'un des boîtiers externes de sorte qu'une extrémité distale de la fibre optique à déclenchement longitudinal soit exposée à travers l'un des boîtiers externes.

6. Effecteur terminal selon la revendication 1, dans lequel les plaques d'étanchéité sont électroconductrices et sont configurées pour une excitation bipolaire afin de sceller un tissu.
